(19) Europäisches Patentamt
European Patent Office
Office européen des brevets

(11) **EP 1 973 527 B1**

(12) **EUROPEAN PATENT SPECIFICATION**

(45) Date of publication and mention
of the grant of the patent:
**07.10.2009 Bulletin 2009/41**

(51) Int Cl.:
***A61K 9/14*** *(2006.01)*

(21) Application number: **06840263.5**

(86) International application number:
**PCT/US2006/062094**

(22) Date of filing: **14.12.2006**

(87) International publication number:
**WO 2007/070852 (21.06.2007 Gazette 2007/25)**

(54) **PROCESSES FOR MAKING PARTICLE-BASED PHARMACEUTICAL FORMULATIONS FOR PARENTERAL ADMINISTRATION**

VERFAHREN ZUR HERSTELLUNG VON PHARMAZEUTISCHEN FORMULIERUNGEN AUF TEILCHENBASIS ZUR PARENTERALEN VERABREICHUNG

PROCÉDÉS DE PRÉPARATION DE FORMULATIONS PHARMACEUTIQUES À BASE DE PARTICULES DESTINÉES À UNE ADMINISTRATION PARENTÉRALE

(84) Designated Contracting States:
**AT BE BG CH CY CZ DE DK EE ES FI FR GB GR HU IE IS IT LI LT LU LV MC NL PL PT RO SE SI SK TR**

(30) Priority: **15.12.2005 US 750461 P**

(43) Date of publication of application:
**01.10.2008 Bulletin 2008/40**

(73) Proprietor: **Acusphere, Inc.**
**Watertown, MA 02472 (US)**

(72) Inventors:
• **ALTREUTER, David**
**Wayland, MA 01778 (US)**
• **BERNSTEIN, Howard**
**Cambridge, MA 02138 (US)**
• **BRITO, Luis**
**Winchester, MA 01890 (US)**
• **BRITO, Shaina**
**Winchester, MA 01890 (US)**

• **CARNEIRO, Olinda, C.**
**Cambridge, MA 02141-1421 (US)**
• **CHICKERING, Donald, E.**
**Framingham, MA 01701 (US)**
• **HUANG, Eric, K.**
**Cambridge, MA 02141 (US)**
• **JAIN, Rajeev**
**Framingham, MA 01701 (US)**
• **NARASIMHAN, Sridhar**
**Elgin, IL 60123 (US)**
• **PANDIT, Namrata**
**Watertown, MA 02472 (US)**
• **STRAUB, Julie, A.**
**Winchester, MA 01890 (US)**

(74) Representative: **HOFFMANN EITLE**
**Patent- und Rechtsanwälte**
**Arabellastrasse 4**
**81925 München (DE)**

(56) References cited:
**WO-A-20/04064752      US-A1- 2003 082 236**
**US-A1- 2004 121 003**

**Description**

**Background of the Invention**

This invention is generally in the field of pharmaceutical compositions comprising particles, such as microparticles, and more particularly to methods for making particulate blend formulations for parenteral administration.

Microparticles comprising therapeutic and diagnostic agents are known to be useful for enhancing the controlled delivery of such agents to humans or animals. For these applications, microparticles having very specific sizes and size ranges are needed in order to effectively deliver these agents. Many drug formulations are produced in a dry powder form for subsequent dispersion or dissolution in liquid media, such as a vehicle for intravascular, subcutaneous, or intramuscular injection.

For a parenteral dosage form of therapeutic microparticles, the microparticles desirably are easily dispersed or dissolved in the liquid media. However, microparticles, particularly those consisting of poorly water soluble pharmaceutical agents, tend to be poorly wettable or poorly dispersible in aqueous media. This may undesirably alter the microparticle formulation's performance, safety and/or reproducibility. Dispersibility and wettability depend on a variety of factors, including the materials and methods used in making the microparticles, the surface (i.e., chemical and physical) properties of the microparticles, and the composition of the suspending medium or vehicle.

One conventional response to the problem of poor dispersibility is the addition of viscosity enhancers or other excipients to the liquid media. However, these additives may have undesirable effects on patients receiving an injection of the suspension. It would be highly desirable to improve microparticle performance (e.g., dispersibility, syringeability, wettability, etc.) and consequently increase the reliability of actual dose of drug delivered, without the use of undesirable additives in the liquid media. It would therefore be useful to provide a process that creates microparticle formulations that are easily wettable and dispersible. Such a manufacturing process should be simple and operate at conditions to minimize equipment and operating costs and to avoid degradation of the pharmaceutical agent.

Excipients often are added to the microparticles and pharmaceutical agents in order to provide the microparticle formulations with certain desirable properties or to enhance processing of the microparticle formulations. For example, the excipients can facilitate administration of the microparticles, minimize microparticle agglomeration upon storage or upon reconstitution, facilitate appropriate release or retention of the active agent, and/or enhance shelf life of the product. Representative types of these excipients include osmotic agents, bulking agents, surfactants, preservatives, wetting agents, pharmaceutically acceptable carriers, and diluents. It is important that the process of combining these excipients and microparticles yield a uniform blend. Combining these excipients with the microparticles can complicate production and scale-up; it is not a trivial matter to make such uniform microparticle pharmaceutical formulations, particularly on a commercial scale.

Furthermore, certain desirable excipient materials are difficult to mill or blend with pharmaceutical agent microparticles. For example, excipients characterized as liquid, waxy, non-crystalline, or non-friable are not readily blended uniformly with drug containing particles and/or are not readily processed through a mill. Conventional dry blending of such materials may not yield the uniform, intimate mixtures of the components, which pharmaceutical formulations require. For example, dry powder formulations therefore should not be susceptible to batch-to-batch or intra-batch compositional variations. Rather, production processes for a pharmaceutical formulation must yield consistent and accurate dosage forms. Such consistency in a dry powder formulation may be difficult to achieve with an excipient that is not readily blended or milled. It therefore would be desirable to provide methods for making uniform blends of microparticles and difficult to blend excipients. Such methods desirably would be adaptable for efficient, commercial scale production.

It therefore would be desirable to provide improved methods for making dry powder blended particle or microparticle pharmaceutical formulations for parenteral administration that have improved wettability and dispersibility upon combination with a liquid vehicle for injection.

**Summary of the Invention**

Methods are provided for making a pharmaceutical particle blend formulation for parenteral administration. In one embodiment, the method includes the steps of (a) providing particles which comprise a pharmaceutical agent; (b) blending the particles with particles of at least one bulking agent to form a first powder blend, which does not include a surfactant; (c) milling the first powder blend to form a milled blend which comprises microparticles or nanoparticles of the pharmaceutical agent; and (d) reconstituting the milled blend with a liquid vehicle suitable for parenteral administration, wherein the vehicle comprises at least one surfactant. In another embodiment, the method includes the steps of: (a) providing particles which comprise a pharmaceutical agent; (b) blending the particles of pharmaceutical agent with particles of at least one excipient to form a first blend; and (c) milling the first blend to form a milled blend which comprises microparticles or nanoparticles, wherein the milled blend exhibits greater dispersibility or suspendability as compared to the particles of step (a) or the first blend. The milling of step (c) may be a jet milling process. The particles of step (a) may be microparticles. The particles of step (a) may further include a polymer. The bulking agent, or the excipient, may include at least one sugar, sugar alcohol, starch, amino acid, or combination thereof. Examples of suitable bulking agents include lactose, sucrose, maltose, mannitol, sorbitol, trehalose, galactose, xylitol, erythritol, and combinations thereof. The liquid vehicle may further comprise a polymer.

In another aspect, a method is provided for making a pharmaceutical formulation for parenteral administration that includes the steps of (a) providing particles which comprise a pharmaceutical agent; and (b) blending the particles of step (a) with particles of a pre-processed excipient to form a dry powder blend, wherein the pre-processed excipient is prepared by (i) dissolving a bulking agent and at least one non-friable excipient in a solvent to form an excipient solution, and (ii) removing the solvent from the excipient solution to form the pre-processed excipient in dry powder form, wherein the dry powder blend can be reconstituted in an aqueous vehicle suitable for parenteral administration. The step of removing the solvent may include spray drying or lyophilization. In one embodiment, the dry powder blend does not include a surfactant. In one embodiment, the method further includes, as a step (c), reconstituting the dry powder blend with a vehicle suitable for parenteral administration. In an alternative embodiment, the method further includes, as a step (c), milling the dry powder blend to form a milled pharmaceutical formulation blend, which comprises microparticles or nanoparticles of the pharmaceutical agent, and optionally, as a step (d), reconstituting the dry powder blend with a vehicle suitable for parenteral administration. The particles of pre-processed excipient may be microparticles or nanoparticles, or both. Again, the bulking agent may include at least one sugar, sugar alcohol, starch, amino acid, or combination thereof. The non-friable excipient may be a liquid, waxy, or non-crystalline compound. In one embodiment, the nan-friable excipient comprises a surfactant, particularly a waxy or liquid surfactant. Examples of non-friable excipients include polyvinylpyrrolidones, polyoxyethylene sorbitan fatty acid esters, or a combination thereof.

In another aspect, pharmaceutical formulations made by any of the foregoing methods are provided. In one embodiment, a parenteral dosage form of a pharmaceutical formulation is provided that includes a liquid suspension of a milled blend of microparticles or nanoparticles of a pharmaceutical agent blended with particles of at least one excipient, dispersed in a pharmaceutically acceptable liquid vehicle for injection. In one particular embodiment, the milled blend does not include a surfactant, and optionally the pharmaceutically acceptable liquid vehicle includes an aqueous solution of a surfactant. In one embodiment, the pharmaceutical agent has a solubility in water of less than 10 mg/mL at 25 °C. The excipient particles may include at least one sugar, sugar alcohol, starch, amino acid, or combination thereof. In one embodiment, the excipient particles include a pre-processed excipient that comprises a bulking agent and at least one non-friable excipient. In one embodiment, the microparticles or nanoparticles of pharmaceutical agent in the milled blend have a volume average diameter of less than 10 $\mu$m. The microparticles or nanoparticles of a pharmaceutical agent may further include a polymer.

**Brief Description of the Drawings**

FIG. 1 is a process flow diagram of one embodiment of a process for making a parenteral dosage form of a pharmaceutical blend formulation.

FIG. 2 is a process flow diagram of one embodiment of a process for making a parenteral dosage form of a pharmaceutical blend formulation that includes a milled dry powder blend of a drug and a pre-processed excipient as described herein.

FIG. 3 is a process flow diagram of one embodiment of a process for pre-processing a non-friable excipient into a dry powder form.

FIGS. 4A-B are light microscope images of celecoxib particles reconstituted from a jet milled blend of celecoxib and non-pre-processed excipients.

FIGS. 5A-B are light microscope images of celecoxib particles reconstituted from a jet milled blend of celecoxib and pre-processed excipients.

FIGS. 6A-B are light microscope images of reconstituted celecoxib from a blend of excipient particles and celecoxib particles.

FIGS. 7A-B are light microscope images of reconstituted celecoxib from a blend of excipient particles and milled celecoxib particles.

FIGS. 8A-B are light microscope images of reconstituted celecoxib from a jet milled blend of excipient particles and celecoxib particles.

FIG. 9 is a light microscope image of reconstituted oxcarbazepine from a jet milled blend of excipient particles pre-processed with surfactant and oxcarbazepine particles.

FIG. 10 is a light microscope image of reconstituted oxcarbazepine from a jet milled blend of excipient particles and oxcarbazepine particles, reconstituted with water only.

**FIG. 11** is a light microscope image of reconstituted oxcarbazepine from a jet milled blend of excipient particles and oxcarbazepine particles, reconstituted with water containing Tween80.

## Detailed Description of the Invention

Processing methods have been developed for making a parenteral dosage form of a pharmaceutical formulation, providing improved dispersibility, suspendability, wettability, and syringeability characteristics, which advantageously can improve the reliability of the actual dose of drug that is delivered to (injected into) the patient. The processes are particularly useful for making parenteral formulations of poorly water-soluble pharmaceutical agents, or poorly water-soluble matrices having dispersed therein a pharmaceutical agent for release, which may provide better bioavailability and/or quicker time to onset of therapeutic effect. The processes may beneficially provide more controlled drug particle size in suspension for better syringeability, enhanced safety of intravenous delivered microparticles and increased reliability of actual dose of drug delivered.

It has been found that dry powder blend formulations exhibiting better wettability or dispersibility may be obtained by the ordered steps of blending particles of pharmaceutical agent with an excipient and then milling the resulting blend, as compared to blends prepared without this combination of steps. Without being limited to any particular theory, it is believed that the criticality of the order is because it allows the surfaces of drug particles to be exposed (either through breakage of drug particles or through deaggregation of drug particle-drug particle agglomerates during the milling process) which permits the excipient particles present to interact with the newly exposed drug particle surfaces, thus leading to decreased drug particle-drug particle interactions.

It has also been beneficially discovered that certain useful but difficult-to-mill, or difficult- to-blend, excipient materials can be used in the process if they are themselves first subjected to a "pre-processing" treatment that transforms the liquid, waxy, or otherwise non-friable excipient into a dry powder form that is suitable for blending and milling in a dry powder form. By milling after blending, it was found that the dry powder blend advantageously has better pharmaceutical agent particle-to-pharmaceutical agent particle contact, thereby providing a blend that is more readily or more rapidly wettable and dispersible. Thus, more uniform and reproducible suspensions may be produced.

As used herein, the term "dispersibility" includes the suspendability of a powder (e.g., a quantity or dose of microparticles) within a liquid. Accordingly, the term "improved dispersibility" refers to a reduction of particle-particle interactions of the microparticles of a powder within a liquid. Improvements in dispersibility can be evaluated using methods that examine the increase in concentration of suspended particles or a decrease in agglomerates. These methods include visual evaluation for turbidity of the suspension, direct turbidity analysis using a turbidimeter or a visible spectrophotometer, light microscopy for evaluation of concentration of suspended particles and/or concentration of agglomerated particles, or Coulter counter analysis for particle size and concentration in suspension or light scattering methods for analysis of particle size in suspension. An increase in turbidity, an increase in the concentration of suspended particles, a decrease in the concentration or size of agglomerated particles, or a decrease in the particle size in suspension based on a volume mean indicates an improvement in dispersibility. Improvements in wettability of the powder can be assessed using contact angle measurements.

The pharmaceutical formulations made as described herein are intended to be administered to a patient (i.e., human or animal in need of the pharmaceutical agent) to deliver an effective amount of a therapeutic, diagnostic, or prophylactic agent. As used herein, "parenteral administration" refers to administration by injection via any of the parenteral routes into essentially any organ or area of the body, including but not limited to intramuscular, intravenous, subcutaneous, intradermal, intraarticular, intrasynovial, or intrathecal administration.

As used herein, the terms "comprise," "comprising," "include," and "including" are intended to be open, non-limiting terms, unless the contrary is expressly indicated.

## The Methods

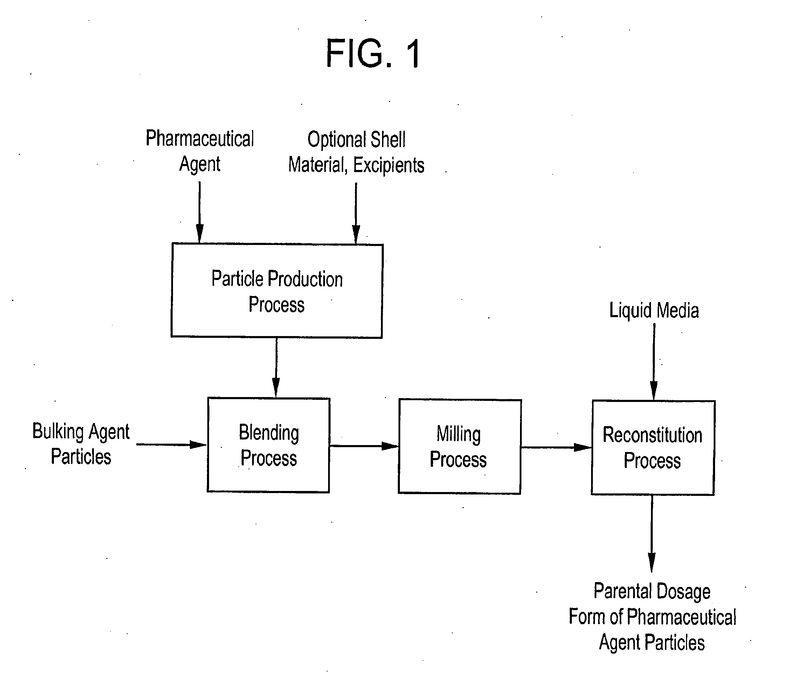

In one embodiment, the method for making a parenteral dosage form of a pharmaceutical agent includes the steps of (a) providing particles which comprise a pharmaceutical agent; (b) blending the particles with particles of at least one bulking agent to form a first powder blend, which does not include a surfactant; (c) milling the first powder blend to form a milled blend which comprises microparticles or nanoparticles of the pharmaceutical agent; and (d) reconstituting the milled blend with a liquid vehicle suitable for parenteral administration, wherein the vehicle comprises at least one surfactant. See **FIG. 1**. In a preferred embodiment, the milling of step (c) is jet milling. In various embodiments, the particles of step (a) may be microparticles, may include a pharmaceutical agent having a solubility in water of less than 10 mg/mL at 25 °C, and may further include a polymer or shell material. The bulking agent may comprise at least one sugar, sugar alcohol, starch, amino acid, or combination thereof. The bulking agent may comprise a pharmaceutically acceptable buffering agent such as a sodium or potassium salt of an acid such as phosphoric acid, acetic acid, citric acid, lactic acid, tartaric acid, succinic acid, or gluconic acid. Examples of suitable bulking agents include lactose, sucrose, maltose, mannitol, sorbitol, trehalose, galactose, xylitol, erythritol, and combinations thereof. The liquid vehicle may be a pharmaceutically acceptable aqueous solution, and optionally may further include a polymer with the surfactant.

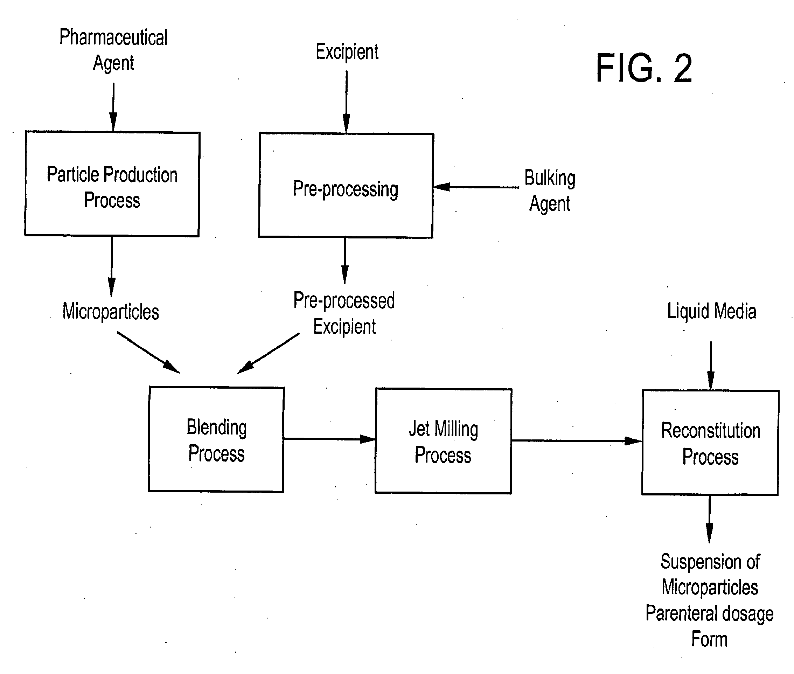

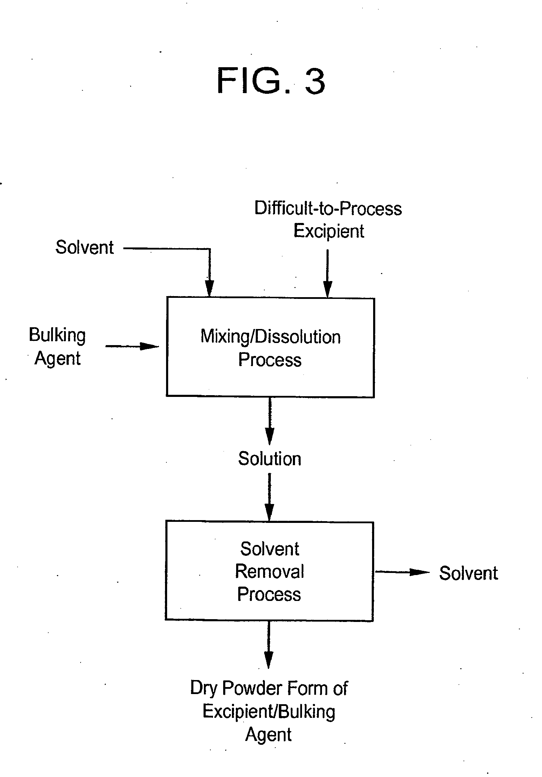

In another embodiment, the method for making a pharmaceutical formulation for parenteral administration, includes the steps of: (a) providing particles which comprise a pharmaceutical agent; and (b) blending the particles of step (a) with particles of a pre-processed excipient to form a dry powder blend, wherein the pre-processed excipient is prepared by (i) dissolving a bulking agent and at least one non-friable excipient in a solvent to form an excipient solution, and (ii) removing the solvent from the excipient solution to form the pre-processed excipient in dry powder form, wherein the dry powder blend can be reconstituted in an aqueous vehicle suitable for parenteral administration. See **FIG. 2** and **FIG. 3**. In various embodiments, the step of removing the solvent may include spray-drying, vacuum drying, or lyophilization. In one embodiment, the method further includes, as a step (c), reconstituting the dry powder blend with a vehicle suitable for parenteral administration. In an alternative embodiment, the method further includes, as a step (c), milling the dry powder blend to form a milled pharmaceutical formulation blend, which comprises microparticles or nanoparticles of the pharmaceutical agent. In one particular variation of this embodiment, the method further includes, as a step (d), reconstituting the dry powder blend with a vehicle suitable for parenteral administration. In one embodiment, the dry powder blend does not include a surfactant. In various embodiments, the pharmaceutical agent particles of step (a), the pre-processed excipient particles of step (b), or both, are microparticles. In various embodiments, the pharmaceutical agent particles of step (a), the pre-processed excipient particles of step (b), or both, are nanoparticles. The bulking agent may comprise at least one sugar, sugar alcohol, starch, amino acid, or combination thereof. Examples of suitable bulking agents include lactose, sucrose, maltose, mannitol, sorbitol, trehalose, galactose, xylitol, erythritol, and combinations thereof. The non-friable excipient may be or include a liquid, waxy, or non-crystalline compound. In one embodiment, the non-friable excipient comprises a surfactant, such as a waxy or liquid surfactant. Non-limiting examples of possible non-friable excipients include polyvinylpyrrolidones, polyoxyethylene sorbitan fatty acid esters, or combinations thereof. In one embodiment, the pharmaceutical agent has a solubility in water of less than 10 mg/mL at 25 °C.

In another aspect, a method is provided for making a pharmaceutical formulation for parenteral administration, comprising the steps of: (a) providing particles which comprise a pharmaceutical agent; (b) blending the particles of pharmaceutical agent with particles of at least one excipient to form a first blend; and (c) milling the first blend to form a milled blend which comprises microparticles or nanoparticles; wherein the milled blend exhibits a greater dispersibility or suspendability as compared to the particles of step (a) or the first blend that results from step (b).

The processes described herein generally can be conducted using batch, continuous, or semi-batch methods. These processes described herein optionally may further include separately milling some or all of the components (e.g., pharmaceutical agent particles, excipient particles) of the blended formulation before they are blended together. In preferred embodiments, the excipient and pharmaceutical agent are in a dry powder form.

Particle Production

The skilled artisan can envision many ways of making particles useful for the methods and formulations described herein, and the following examples describing how particles may be formed or provided are not intended to limit in any way the methods and formulations described and claimed herein. The particles comprising pharmaceutical agent that are used or included in the methods and formulations described herein can be made using a variety of techniques known in the art. Suitable techniques may include solvent precipitation, crystallization, spray drying, melt extrusion, compression molding, fluid bed drying, solvent extraction, hot melt encapsulation, phase inversion encapsulation, and solvent evaporation.

For instance, the microparticles may be produced by crystallization. Methods of crystallization include crystal formation upon evaporation of a saturated solution of the pharmaceutical agent, cooling of a hot saturated solution of the pharmaceutical agent, addition of antisolvent to a solution of the pharmaceutical agent (drowning or solvent precipitation), pressurization, addition of a nucleation agent such as a crystal to a saturated solution of the pharmaceutical agent, and contact crystallization (nucleation initiated by contact between the solution of the pharmaceutical agent and another item such as a blade).

Another way to form the particles, preferably microparticles, is by spray drying. See, e.g., U.S. Patents No. 5,853,698 to Straub et at.; No. 5,611,344 to Bernstein et al.; No. 6,395,300 to Straub ct al.; and No. 6,223,455 to Chickering III et al. As defined herein, the process of "spray drying" a solution containing a pharmaceutical agent and/or shell material refers to a process wherein the solution is atomized to form a fine mist and dried by direct contact with hot carrier gases. Using spray drying equipment available in the art, the solution containing the pharmaceutical agent and/or shell material may be atomized into a drying chamber, dried within the chamber, and then collected via a cyclone at the outlet of the chamber. Representative examples of types of suitable atomization devices include ultrasonic, pressure feed, air atomizing, and rotating disk. The temperature may be varied depending on the solvent or materials used. The temperature ofthe inlet and outlet ports can be controlled to produce the desired products. The size of the particulates of pharmaceutical agent and/or shell material is a function of the nozzle used to spray the solution of pharmaceutical agent and/or shell material, nozzle pressure, the solution and atomization flow rates, the pharmaceutical agent and/or shell material used, the concentration of the pharmaceutical agent and/or shell material, the type of solvent, the temperature of spraying (both inlet and outlet temperature), and the molecular weight of a shell material such as a polymer or other matrix material.

A further way to make the particles is through the use of solvent evaporation, such as described by Mathiowitz et al., J. Scanning Microscopy, 4:329 (1990); Beck et al., Fertil. Steril, 31:545 (1979) and Benita et al., J. Pharm. Sci., 73:1721 (1984). In still another example, hot-melt microencapsulation is used, such as described in Mathiowitz, et al., Reactive Polymers, 6:275 (1987). In another example, a phase inversion encapsulation may be used, such as described in U.S. Patent No. 6,143,211 to Mathiowitz et al. This causes a phase inversion and spontaneous formation of discrete microparticles, typically having an average particle size of between 10 nm and 10 $\mu$m.

In yet another approach, a solvent removal technique may be used, wherein a solid or liquid pharmaceutical agent is dispersed or dissolved in a solution of a shell material in a volatile organic solvent and the mixture is suspended by stirring in an organic oil to form an emulsion. Unlike solvent evaporation, however, this method can be used to make microparticles from shell materials such as polymers with high melting points and different molecular weights. The external morphology of particles produced with this technique is highly dependent on the type of shell material used.

In another approach, an extrusion technique may be used to make microparticles of shell materials. For example, such microparticles may be produced by dissolving the shell material (e.g., gel-type polymers, such as polyphosphazene or polymethylmethacrylate) in an aqueous solution, homogenizing the mixture, and extruding the material through a microdroplet forming device, producing microdroplets that fall into a slowly stirred hardening bath of an oppositely charged ion or polyelectrolyte solution.

Pre-Processing the Excipient

When it is necessary or desirable to convert a liquid, waxy, or otherwise non-friable excipient into a dry powder form suitable for blending and milling, these difficult-to-mill and difficult-to-blend excipient materials are "pre-processed." In preferred embodiments, the pre-processed excipient that is used or included in the methods and formulations described herein is prepared by (i) dissolving a bulking agent and at least one non-friable excipient in a solvent to form an excipient solution, and then (ii) removing the solvent from the excipient solution to form the pre-processed excipient in dry powder form. See **FIG. 3.** The dissolution of bulking agent and at least one non-friable excipient in a solvent can be done simply by mixing appropriate amounts of these three components together in any order to form a well-mixed solution. A variety of suitable methods of solvent removal known in the art may be used in this process. In one embodiment, the step of removing the solvent comprises spray drying. In another embodiment, the step of removing the solvent comprises lyophilization, vacuum drying, or freeze drying. The pre-processed excipient in dry powder form optionally may be milled prior to blending with the particles comprising pharmaceutical agent.

In yet another variation of preparing pre-processed excipient particles, the particles of at least one excipient are made by mixing a dry powder bulking agent with at least one non-friable excipient. The at least one non-friable excipient may be in an essentially dry particle form or may be dissolved in a solvent at the time the at least one non-friable excipient is mixed with the bulking agent. Residual solvent is removed to form the pre-processed excipient particles.

It is contemplated that the particles of pharmaceutical agent (e.g., active pharmaceutical ingredient microparticles) can be blended with one or more pre-processed excipients, and optionally, can be combined with one or more excipients that have not been pre-processed. The particles can be blended with pre-processed excipient(s) either before or after blending with excipient(s) that have not been pre-processed. One or more of the excipients may be milled prior to combining with the pharmaceutical agent microparticles.

Blending and Milling

The particles of pharmaceutical agent are blended with one or more other excipient particulate materials, in one or more steps, and then the resulting blend is milled. Content uniformity of solid-solid pharmaceutical blends is critical. Comparative studies indicate that the milling of a blend (drug plus excipient) can yield a dry powder pharmaceutical formulation that exhibits improved wettability and/or dispersibility as compared to a formulation made by milling and then blending or by blending without milling. That is, the sequence of the two steps is important to the performance of the ultimate parenteral dosage form. In a preferred embodiment, pharmaceutical agent microparticles are blended with one or more excipients of interest, and the resulting blend is then jet milled to yield a uniform mixture of microparticles and excipient.

1. Blending

The skilled artisan can envision many ways of blending particles in and for the methods and formulations described herein, and the following examples describing how particles may be blended are not intended to limit in any way the methods and formulations described and claimed herein. The blending can be conducted in one or more steps, in a continuous, batch, or semi-batch process. For example, if two or more excipients are used, they can be blended together before, or at the same time as, being blended with the pharmaceutical agent microparticles.

The blending can be carried out using essentially any technique or device suitable for combining the microparticles with one or more other materials (e.g., excipients) effective to achieve uniformity of blend. The blending process may be performed using a variety of blenders. Representative examples of suitable blenders include V-blenders, slant-cone blenders, cube blenders, bin blenders, static continuous blenders, dynamic continuous blenders, orbital screw blenders, planetary blenders, Forberg blenders, horizontal double-arm blenders, horizontal high intensity mixers, vertical high intensity mixers, stirring vane mixers, twin cone mixers, drum mixers, and tumble blenders. The blender preferably is of a strict sanitary design required for pharmaceutical products.

Tumble blenders are often preferred for batch operation. In one embodiment, blending is accomplished by aseptically combining two or more components (which can include both dry components and small portions of liquid components) in a suitable container. One example of a tumble blender is the TURBULA™, distributed by Glen Mills Inc., Clifton, NJ, USA, and made by Willy A. Bachofen AG, Maschinenfabrik, Basel, Switzerland.

For continuous or semi-continuous operation, the blender optionally may be provided with a rotary feeder, screw conveyor, or other feeder mechanism for control led introduction of one or more of the dry powder components into the blender.

2. <u>Milling</u>

The milling step is used to fracture and/or deagglomerate the blended particles, to achieve a desired particle size and size distribution, as well as to enhance distribution of the particles within the blend. The skilled artisan can envision many ways of milling particles or blends in the methods and formulations described herein, and the following examples describing how such particles or blend may be milled are not intended to limit in any way the methods and formulations described and claimed herein. A variety of milling processes and equipment known in the art may be used. Examples include hammer mills, ball mills, roller mills, disc grinders and the like. Preferably, a dry milling process is used.

In a preferred technique, the milling comprises jet milling. Jet milling is described for example in U.S. Patent No. 6,962,006 to Chickering III et al. As used herein, the terms "jet mill" and "jet milling" include and refer to the use of any type of fluid energy impact mills, including spiral jet mills, loop jet mills, and fluidized bed jet mills, with or without internal air classifiers. In one embodiment, the jet milling process conditions are selected so that the size and morphology of the individual microparticles following milling has a volume average size reduction of at least 15% and a number average size reduction of no more than 75%. In one embodiment, particles are aseptically fed to the jet mill via a feeder, and a suitable gas, preferably dry nitrogen, is used to feed and grind the microparticles through the mill. Grinding and feed gas pressures can be adjusted based on the material characteristics. Microparticle throughput depends on the size and capacity of the mill. The milled microparticles can be collected by filtration or, more preferably, cyclone.

<u>Processing Into Parenteral Dosage Form</u>

The skilled artisan can envision several ways of processing the particle blends in the methods and for the formulations described herein, and the following examples describing how parenteral dosage forms may be produced are not intended to limit in any way the methods and formulations described and claimed herein. For injectable dosage forms, the milled dry powder blend may be filled directly into a container (such as a vial) and sealed, and then the dosage form is reconstituted prior to use by adding a reconstitution medium. The resulting microparticle formulation can provide improved injectability, passing through the needle of a syringe more easily. The reconstitution medium is a liquid vehicle suitable for injection and compatible with the milled dry powder blend. The liquid vehicle may be aqueous or non-aqueous vehicles known in the art. Examples of suitable media include water for injection, physiological saline, 5% dextrose, phosphate buffered saline, 5% mannitol, Ringer's Injection, Lactated Ringer's Injection, 5% dextrose in Lactated Ringer's Injection, bacteriostatic water for injection, bacteriostatic saline, 10% dextrose in water, 10% mannitol in water, 6% dextran 5% dextrose, 6% dextran 0.9% sodium chloride, 10% fructose, 5% invert sugar, 1/6 M sodium lactate, parenteral nutritional solutions such as amino acid injection, and parenteral nutritional emulsions such as Intralipid. Examples of non-aqueous vehicles include alcohols, glycerin, n-methyl pyrrolidone (NMP), and fixed vegetable oils. These media may include antibacterial preservatives, buffers, and osmotic agents. The media may include one or more surfactants such as polysorbate 80, polysorbate 20, and a combination thereof.

In one embodiment, a parenteral dosage form of a pharmaceutical formulation is provided which includes a liquid suspension of a milled blend of microparticles or nanoparticles of a pharmaceutical agent blended with particles of at least one excipient, dispersed in a pharmaceutically acceptable liquid vehicle for injection. In one embodiment, the milled blend does not include a surfactant. Optionally, the pharmaceutically acceptable liquid vehicle comprises an aqueous solution of a surfactant. In a preferred embodiment, the pharmaceutical agent has a solubility in water of less than 10 mg/mL at 25 °C. In various embodiments, the excipient particles comprise at least one sugar, sugar alcohol, starch, amino acid, or combination thereof. In one embodiment, the excipient particles include a pre-processed excipient which comprises a bulking agent and at least one non-friable excipient. In another embodiment, the microparticles or nanoparticles of a pharmaceutical agent further comprise a polymer, such as a biodegradable or bioerodible synthetic polymer known in the art.

The milled blend may optionally undergo additional processes before being finally made into a parenteral dosage form. Representative examples of such processes include lyophilization or vacuum drying to further remove residual solvents, temperature conditioning to anneal materials, size classification to recover or remove certain fractions of the particles (i.e., to optimize the size distribution), granulation, and sterilization. In one embodiment, the milled blend is further blended with one or more additional dry powder excipient materials (e.g., excipients microparticles), which may be the same as or different from the excipient material(s) in the milled blend.

## The Particles and Formulation Components

The parenteral dosage formulations made as described herein include mixtures of particles. The mixture generally includes (1) microparticles or nanoparticles that comprise the pharmaceutical agent and that may optionally comprise a shell material, and (2) particles of at least one, and typically more than one, excipient material.

## Particles

The particles comprising pharmaceutical agent that are provided as a starting material in the methods described herein can be provided in a variety of sizes and compositions. As used herein, the term "particles" includes microparticles and nanoparticles, as well as larger particles, e.g., up to 5 mm in the longest dimension. In a preferred embodiment, the particles are microparticles. As used herein, the term "microparticle" encompasses microspheres and microcapsules, as well as microparticles, unless otherwise specified, and denotes particles having a size of 1 to 1000 microns. As used herein, "nanoparticles" have a size of 1 to 1000 nm. The particles are manufactured to have a size (i.e., diameter) suitable for the intended route of administration. Particle size also can affect RES uptake. In various embodiments, the microparticles or nanoparticles of pharmaceutical agent in the milled pharmaceutical formulation blend have a volume average diameter of less than 100 $\mu$m, preferably less than 20 $\mu$m, more preferably less than 10 $\mu$m. For intravascular administration, the particles preferably have a number average diameter of between 0.5 and 8 $\mu$m. For subcutaneous or intramuscular administration, the particles preferably have a number average diameter of between about 1 and 100 $\mu$m. In one embodiment, the particles of the milled pharmaceutical formulation blend have a volume average diameter of between about 0.5 and 20 $\mu$m.

Microparticles may or may not be spherical in shape. Microparticles can be rod like, sphere like, acicular (slender, needle-like particle of similar width and thickness), columnar (long, thin particle with a width and thickness that are greater than those of an acicular particle), flake (thin, flat particle of similar length and width), plate (flat particle of similar length and width but with greater thickness than flakes), lath (long, thin, blade-like particle), equant (particles of similar length, width, and thickness, this includes both cubical and spherical particles), lamellar (stacked plates), or disc like. "Microcapsules" are defined as microparticles having an outer shell surrounding a core of another material, in this case, the pharmaceutical agent. The core can be gas, liquid, gel, solid, or a combination thereof. "Microspheres" can be solid spheres, can be porous and include a sponge-like or honeycomb structure formed by pores or voids in a matrix material or shell, or can include multiple discrete voids in a matrix material or shell.

In one embodiment, the particle is formed entirely of the pharmaceutical agent. In another embodiment, the particle has a core of pharmaceutical agent encapsulated in a shell. In yet another embodiment, the pharmaceutical agent is interspersed within a shell or matrix. In still another embodiment, the pharmaceutical agent is uniformly mixed within the material comprising the shell or matrix.

The terms "size" or "diameter" in reference to particles refers to the number average particle size, unless otherwise specified. An example of an equation that can be used to describe the number average particle size (and is representative of the method used for the Coulter counter) is shown below:

$$\frac{\sum_{i=1}^{p} n_i d_i}{\sum_{i=1}^{p} n_i}$$

where $n$ = number of particles of a given diameter ($d$).

As used herein, the term "volume average diameter" refers to the volume weighted diameter average. An example of an equation that can be used to describe the volume average diameter, which is representative of the method used for the Coulter counter is shown below:

$$\left[ \frac{\sum_{i=1}^{p} n_i d_i^{3}}{\sum_{i=1}^{p} n_i} \right]^{1/3}$$

where $n$ = number of particles of a given diameter ($d$).

Another example of an equation that can be used to describe the volume mean, which is representative of the equation used for laser diffraction particle analysis methods, is shown below:

$$\frac{\sum d^{4}}{\sum d^{3}}$$

where d represents diameter. When a Coulter counter method is used, the raw data is directly converted into a number based distribution, which can be mathematically transformed into a volume distribution. When a laser diffraction method is used, the raw data is directly converted into a volume distribution, which can be mathematically transformed into a number distribution.

In the case of a non-spherical particle, the particles can be analyzed using Coulter counter or laser diffraction methods, with the raw data being converted to a particle size distribution by treating the data as if it came from spherical particles. If microscopy methods are used to assess the particle size for non-spherical particles, the longest axis can be used to represent the diameter (d), with the particle volume ($V_p$) calculated as:

$$V_p = \frac{4\pi r^{3}}{3}$$

where r is the particle radius (0.5d),
and a number mean and volume mean are calculated using the same equations used for a Coulter counter.

Particle size analysis can be performed on a Coulter counter, by light microscopy, scanning electron microscopy, transmission electron microscopy, laser diffraction methods, light scattering methods or time of flight methods. Where a Coulter counter method is described, the powder is dispersed in an electrolyte, and the resulting suspension analyzed using a Coulter Multisizer II fitted with a 50-$\mu$m aperture tube. Where a laser diffraction method is used, the powder is dispersed in an aqueous medium and analyzed using a Coulter LS230, with refractive index values appropriately chosen for the material being tested.

Analysis for agglomerates can be performed by visual evaluation of a suspension for the presence of macroscopic agglomerates, light microscopy for concentration of microscopic agglomerates, Coulter counter analysis or light scattering methods of analysis for particle size in suspension. A decrease in the particle size in suspension based on a volume mean indicates a decreased level of agglomerates.

1. Pharmaceutical Agent

The pharmaceutical agent is a therapeutic, diagnostic, or prophylactic agent. It may be an active pharmaceutical ingredient ("API"), and may be referred to herein generally as a "drug" or "active agent." The pharmaceutical agent may be present in an amorphous state, a crystalline state, or a mixture thereof. The pharmaceutical agent may be labeled with a detectable label such as a fluorescent label, radioactive label or an enzymatic or chromatographically detectable agent

The methods described herein advantageously can be used with pharmaceutical agents having low aqueous solubility, for example, where the pharmaceutical agent has a solubility in water of less than 10 mg/mL at 25 °C.

The methods can be applied to a wide variety of therapeutic, diagnostic and prophylactic agents that may be suitable for parenteral administration. Representative examples of suitable drugs include the following categories and examples of drugs and alternative forms of these drugs such as alternative salt forms, free acid forms, free base forms, and hydrates:

analgesics/antipyretics (e.g., aspirin, acetaminophen, ibuprofen, naproxen sodium, buprenorphine, propoxyphene hydrochloride, propoxyphene napsylate, meperidine hydrochloride, hydromorphone hydrochloride, morphine, oxycodone, codeine, dihydrocodeine bitartratc, pentazocine, hydrocodonc bitartrate, levorphanol, diflunisal, trolamine salicylate, nalbuphine hydrochloride, mefenamic acid, butorphanol, choline salicylate, butalbital, phonyltoloxamine citrate, and meprobaniate);

antiasthmatics;

antibiotics (e.g., neomycin, streptomycin, chloramphenicol, cephalosporin, ampicillin, penicillin, tetracycline, and ciprofloxacin);

antidepressants (e.g., nefopam, oxypertine, doxepin, amoxapine, trazodone, amitriptyline, maprotiline, phenelzine, desipramine, nortriptyline, tranylcypromine, fluoxetine, imipramine, imipramine pamoate, isocarboxazid, trimipramine, and protriptyline);

antidiabetics (e.g., biguanides and sulfonylurea derivatives);

antifungal agents (e.g., griseofulvin, ketoconazole, itraconizole, virconazole, amphotericin B, nystatin, and candicidin);

antihypertensive agents (e.g., propanolol, propafenone, oxyprenolol, nifedipine, reserpine, trimethaphan, phenoxybenzamine, pargyline hydrochloride, deserpidine, diazoxide, guanethidine monosulfate, minoxidil, rescinnamine, sodium nitroprusside, rauwolfia serpentina, alseroxylon, and phentolamine);

anti-inflammatories (e.g., (non-steroidal) celecoxib, rofecoxib, indomethacin, ketoprofen, flurbiprofen, naproxen, ibuprofen, ramifenazone, piroxicam, (steroidal) cortisone, dexamethasone, fluazacort, hydrocortisone, prednisolone, and prednisone);

antineoplastics (e.g., cyclophosphamide, actinomycin, bleomycin, daunorubicin, doxorubicin, epirubicin, mitomycin, methotrexate, fluorouracil, carboplatin, carmustine (BCNU), methyl-CCNU, cisplatin, antiapoptotic agents, etoposide, camptothecin and derivatives thereof, phenesterine, paclitaxel and derivatives thereof, docetaxel and derivatives thereof, vinblastine, vincristine, tamoxifen, and piposulfan);

antianxiety agents (e.g., lorazepam, buspirone, prazepam, chlordiazepoxide, oxazepam, clorazepate dipotassium, diazepam, hydroxyzine pamoate, hydroxyzine hydrochloride, alprazolam, droperidol, halazepam, chlormezanone, and dantrolene);

immunosuppressive agents (e.g., cyclosporine, azathioprine, mizoribine, and FK506 (tacrolimus), sirolimus);

antimigraine agents (e.g., ergotamine, propanolol, and dichloralphenazone); sedatives/hypnotics (e.g., barbiturates such as pentobarbital, pentobarbital, and secobarbital; and benzodiazapines such as flurazepam hydrochloride, and triazolam);

antianginal agents (e.g., beta-adrenergic blockers; calcium channel blockers such as nifedipine, and diltiazem; and nitrates such as nitroglycerin, and erythrityl tetranitrate);

antipsychotic agents (e.g., haloperidol, loxapine succinate, loxapine hydrochloride, thioridazine, thioridazine hydrochloride, thiothixene, fluphenazine, fluphenazine decanoate, fluphenazine enanthate, trifluoperazine, lithium citrate, prochlorperazine, aripiprazole; and risperdione);

antimanic agents (e.g., lithium carbonate);

antiarrhythmics (e.g., bretylium tosylate, esmolol, verapamil, amiodarone, encainide, digoxin, digitoxin, mexiletine, disopyramide phosphate, procainamide, quinidine sulfate, quinidine gluconate, flecainide acetate, tocainide, and lidocaine);

antiarthritic agents (e.g., phenylbutazone, sulindac, penicillamine, salsalate, piroxicam, azathioprine, indomethacin, meclofenamate, gold sodium thiomalate, ketoprofen, auranofin, aurothioglucose, and tolmetin sodium);

antigout agents (e.g., colchicine, and allopurinol);

anticoagulants (e.g., heparin, low molecular weight heparin, desirudin, heparin sodium, and warfarin sodium);

thrombolytic agents (e.g., urokinase, streptokinase, and alteplase);

antifibrinolytic agents (e.g., aminocaproic acid);

hemorheologic agents (e.g., pentoxifylline);

antiplatelet agents (e.g., aspirin, clopidogrel);

anticonvulsants (e.g., valproic acid, divalproex sodium, phenytoin, phenytoin sodium, clonazepam, primidone, phenobarbitol, carbamazepine, amobarbital sodium, methsuximide, metharbital, mephobarbital, paramethadione, ethotoin, phenacemide, secobarbitol sodium, clorazepate dipotassium, oxcarbazepine and trimethadione);

antiparkinson agents (e.g., ethosuximide);

antihistamines/antipruritics (e.g., hydroxyzine, diphenhydramine, chlorpheniramine, brompheniramine maleate, cyproheptadine hydrochloride, terfenadine, clemastine fumarate, azatadine, tripelennamine, dexchlorpheniramine maleate, methdilazine);

agents useful for calcium regulation (e.g., calcitonin, and parathyroid hormone);

antibacterial agents (e.g., amikacin sulfate, aztreonam, chloramphenicol, chloramphenicol palmitate, ciprofloxacin, clindamycin, clindamycin palmitate, clindamycin phosphate, metronidazole, metronidazole hydrochloride, gentamicin sulfate, lincomycin hydrochloride, tobramycin sulfate, vancomycin hydrochloride, polymyxin B sulfate, colistimethate sodium, clarithromycin and colistin sulfate);

antiviral agents (e.g., interferons, zidovudine, amantadine hydrochloride, ribavirin, and acyclovir);

antimicrobials (e.g., cephalosporins such as ceftazidime; penicillins; erythromycins; and tetracyclines such as tetracycline hydrochloride, doxycycline byclate, and minocycline hydrochloride, azithromycin, clarithromycin);

anti-infectives (e.g., GM-CSF);

bronchodilators (e.g., sympathomimetics such as epinephrine hydrochloride, metaproterenol sulfate, terbutaline sulfate, isoetharine, isoetharine mesylate, isoetharine hydrochloride, albuterol sulfate, albuterol, bitolterolmesylate, isoproterenol hydrochloride, terbutaline sulfate, epinephrine bitartrate, metaproterenol sulfate, epinephrine, and epinephrine bitartrate; anticholinergic agents such as ipratropium bromide; xanthines such as aminophylline, dyphylline, metaproterenol sulfate, and aminophylline; mast cell stabilizers such as cromolyn sodium; salbutamol; ipratropium bromide; ketotifen; salmeterol; xinafoate; terbutaline sulfate; theophylline; nedocromil sodium; metaproterenol sulfate; albuterol);

corticosteroids (e.g., beclomethasone dipropionate (BDP), beclomethasone dipropionate monohydrate; budesonide, triamcinolone; flunisolide; fluticasone proprionate; mometasone); steroidal compounds and hormones (e.g., androgens such as danazol, testosterone cypionate, fluoxymesterone, ethyltestosterone, testosterone enathate, methyltestosterone, fluoxymesterone, and testosterone cypionate; estrogens such as estradiol, estropipate, and conjugated estrogens; progestins such as methoxyprogesterone acetate, and norethindrone acetate; corticosteroids such as triamcinolone, betamethasone, betamethasone sodium phosphate, dexamethasone, dexamethasone sodium phosphate, prednisone, methylprednisolone acetate suspension, triamcinolone acetonide, methylprednisolone, prednisolone sodium phosphate, methylprednisolone sodium succinate, hydrocortisone sodium succinate, triamcinolone hexacetonide, hydrocortisone, hydrocortisone cypionate, prednisolone, fludrocortisone acetate, paramethasone acetate, prednisolone tebutate, prednisolone acetate, prednisolone sodium phosphate, and hydrocortisone sodium succinate; and thyroid hormones such as levothyroxine sodium);

hypoglycemic agents (e.g., human insulin, purified beef insulin, purified pork insulin, glyburide, chlorpropamide, glipizide, tolbutamide, and tolazamide);

hypolipidemic agents (e.g., clofibrate, dextrothyroxine sodium, probucol, pravastitin, atorvastatin, lovastatin, and niacin);

proteins (e.g., DNase, alginase, superoxide dismutase, and lipase);

nucleic acids (e.g., sense or anti-sense nucleic acids encoding any therapeutically useful protein, including any of the proteins described herein);

agents useful for erythropoiesis stimulation (e.g., erythropoietin);

antiulcer/antireflux agents (e.g., famotidine, cimetidine, and ranitidine hydrochloride);

antinauseants/antiemetics (e.g., meclizine hydrochloride, nabilone, prochlorperazine, dimenhydrinate, promethazine hydrochloride, thiethylperazine, and scopolamine); and

oil-soluble vitamins (e.g., vitamins A, D, E, K, and the like);

as well as other drugs such as mitotane, halonitrosoureas, anthrocyclines, and ellipticine. A description of these and other classes of useful drugs and a listing of species within each class can be found in Martindale, The Extra Pharmacopoeia. 30th Ed. (The Pharmaceutical Press, London 1993).

In a preferred embodiment, the pharmaceutical agent used in the methods and formulations described herein is a hydrophobic compound, particularly a hydrophobic therapeutic agent. Examples of such hydrophobic drugs include clopidogrel, celecoxib, rofecoxib, paclitaxel, docetaxel, acyclovir, alprazolam, amiodaron, amoxicillin, anagrelide, bactrim, biaxin, budesonide, bulsulfan, carbamazepine, ceftazidime, cefprozil, ciprofloxicin, clarithromycin, clozapine, cyclosporine, diazepam, estradiol, etodolac, fameiclovir, fenofibrate, fexofenadine, gemcitabine, ganciclovir, itraconazole, lamotrigine, loratidine, lorazepam, meloxicam, mesalamine, minocycline, modafinil, nabumetone, nelfinavir mesylate, olanzapine, oxcarbazepine, phenytoin, propofol, ritinavir, SN-38, sulfamethoxazol, sulfasalazine, tracrolimus, tiagabine, tizanidine, trimethoprim, valium, valsartan, voriconazole, zafirlukast, zileuton, and ziprasidone.

In particular examples of the methods and formulations described herein, the drug is selected from among clopidogrel, antiepileptics (specifically topiramate and oxcarpazepine), propofol, paclitaxel, docetaxel, celecoxib, and acetaminophen.

Examples of other possible drugs include albuterol, adapalene, doxazosin mesylate, mometasone furoate, ursodiol, amphotericin, enalapril maleate, felodipine, nefazodone hydrochloride, valrubicin, albendazole, conjugated estrogens, medroxyprogesterone acetate, nicardipine hydrochloride, zolpidem tartrate, amlodipine besylate, ethinyl estradiol, omeprazole, rubitecan, amlodipine besylate/ benazepril hydrochloride, etodolac, paroxetine hydrochloride, paclitaxel, atovaquone, felodipine, podofilox, paricalcitol, betamethasone dipropionate, fentanyl, pramipexole dihydrochloride, Vitamin $D_3$ and related analogues, finasteride, quetiapine fumarate, alprostadil, candesartan, cilexetil, fluconazole, ritonavir, busulfan, carbamazepine, flumazenil, risperidone, carbemazepine, carbidopa, levodopa, ganciclovir, saquinavir, amprenavir, carboplatin, glyburide, sertraline hydrochloride, rofecoxib carvedilol, halobetasolproprionate, sildenafil citrate, celecoxib, chlorthalidone, imiquimod, simvastatin, citalopram, ciprofloxacin, irinotecan hydrochloride, sparfloxacin, efavirenz, cisapride monohydrate, lansoprazole, tarnsulosin hydrochloride, mofafinil, clarithromycin, letrozole, terbinafine hydrochloride, rosiglitazone maleate, diclofenac sodium, lomefloxacin hydrochloride, tirofiban hydrochloride, telmisartan, diazapam, loratadine, toremifene citrate, thalidomide, dinoprostone, mefloquine hydrochloride, trandolapril, docetaxel, mitoxantrone hydrochloride, trctinoin, ctodolac, triamcinolone acetate, estradiol, ursodiol, nelfinavir mesylate, indinavir, beclomethasone dipropionate, oxaprozin, flutamide, famotidine, nifedipine, prednisone, cefuroxime, lorazepam, digoxin, lovastatin, griseofulvin, naproxen, ibuprofen, isotretinoin, tamoxifen citrate, nimodipine, amiodarone, and alprazolam.

Additional examples of drugs that may be useful in the methods and formulations described herein include ceftriaxone, ketoconazole, ceftazidime, oxaprozin, albuterol, valacyclovir, urofollitropin, famciclovir, flutamide, enalapril, mefformin, itraconazole, buspirone, gabapentin, fosinopril, tramadol, acarbose, lorazepan, follitropin, glipizide, omeprazole, fluoxetine, lisinopril, tramsdol, levofloxacin, zafirlukast, interferon, growth hormone, interleukin, erythropoietin, granulocyte stimulating factor, nizatidine, bupropion, ' perindopril, erbumine, adenosine, alendronate, alprostadil, benazepril, betaxolol, bleomycin sulfate, dexfenfluramine, diltiazem, fentanyl, flecainid, gemcitabine, glatiramer acetate, granisetron, lamivudine, mangafodipir trisodium, mesalamine, metoprolol fumarate, metronidazole, miglitol, moexipril, monteleukast, octreotide acetate, olopatadine, paricalcitol, somatropin, sumatriptan succinate, tacrine, verapamil, nabumetone, trovafloxacin, dolasetron, zidovudine, finasteride, tobramycin, isradipine, tolcapone, enoxaparin, fluconazole, lansoprazole, terbinafine, pamidronate, didanosine, diclofenac, cisapride, venlafaxine, troglitazone, fluvastatin, losartan, imiglucerase, donepezil, olanzapine, valsartan, fexofenadine, calcitonin, and ipratropium bromide. These drugs are generally considered water-soluble.

In another embodiment, the pharmaceutical agent used in the methods and formulations described herein is a contrast agent for diagnostic imaging. For example, the diagnostic agent may be an imaging agent useful in positron emission tomography (PET), computer assisted tomography (CAT), single photon emission computerized tomography, x-ray, fluoroscopy, magnetic resonance imaging (MRI), or ultrasound imaging. Microparticles loaded with these agents can be detected using standard techniques available in the art and commercially available equipment. Examples of suitable materials for use as MRI contrast agents include soluble iron compounds (ferrous gluconate, ferric ammonium citrate) and gadolinium-diethylenetriaminepentaacetate (Gd-DTPA).

2. <u>Shell Material</u>

The particles that include the pharmaceutical agent may also include a shell material. The shell material can be the same or different from the excipient material. The shell material can be water soluble or water insoluble, degradable or non-degradable, erodible or non-erodible, natural or synthetic, depending for example on the particular parenteral dosage form selected and release kinetics desired. Representative examples of types of shell materials include polymers, amino acids, sugars, proteins, carbohydrates, and lipids. Polymeric shell materials can be degradable or non-degradable, erodible or non-erodible, natural or synthetic. Non-erodible polymers may be used for parenteral administration. In general, synthetic polymers may be preferred due to more reproducible synthesis and degradation. Natural polymers also may be used. A polymer may be selected based on a variety of performance factors, including shelf life, the time required for stable distribution to the site where delivery is desired, degradation rate, mechanical properties, and glass transition temperature of the polymer.

Representative examples of synthetic polymers include poly(hydroxy acids) such as poly(lactic acid), poly (glycolic acid), and poly(lactic acid-co-glycolic acid), poly(lactide), poly(glycolide), poly(lactide-co-glycolide), polyanhydrides, polyorthoesters, polyamides, polyalkylenes such as polyethylene and polypropylene, polyalkylene glycols such as poly(ethylene glycol), polyalkylene oxides such as poly(ethylene oxide), polyvinyl alcohols, polyvinyl ethers, polyvinyl esters, polyvinylpyrrolidone, poly(vinyl alcohols), derivativized celluloses such as alkyl cellulose, hydroxyalkyl celluloses, cellulose ethers, cellulose esters, nitro celluloses, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxyethyl cellulose, cellulose triacetate, and cellulose sulphate sodium salt jointly referred to herein as "synthetic celluloses"), poly(butyric acid), poly(valeric acid), and poly(lactide-co-caprolactone), copolymers and blends thereof. As used herein, "derivatives" include polymers having substitutions, additions of chemical groups, for example, alkyl, alkylene, hydroxylations, oxidations, and other modifications routinely made by those skilled in the art.

Examples of preferred biodegradable polymers include polymers of hydroxy acids such as lactic acid and glycolic acid, and copolymers with polyethylene glycol (PEG), polyanhydrides, poly(ortho)esters, poly(butyric acid), poly (valeric acid), poly(lactide-co-caprolactone), blends and copolymers thereof.

Examples of preferred natural polymers include proteins such as albumin and prolamines, for example, zein, and polysaccharides such as alginate, cellulose and polyhydroxyalkanoates, for example, polyhydroxybutyrate. The *in vivo* stability of the matrix can be adjusted during the production by using polymers such as polylactide-co-glycolide copolymerized with PEG. PEG, if exposed on the external surface, may extend the time these materials circulate post intravascular administration, as it is hydrophilic and has been demonstrated to mask reticuloendothelial system (RES) recognition.

Representative amino acids that can be used in the shell include both naturally occurring and non-naturally occurring amino acids. The amino acids can be hydrophobic or hydrophilic and may be D amino acids, L amino acids or racemic mixtures. Amino acids that can be used include glycine, arginine, histidine, threonine, asparagine, aspartic acid, serine, glutamate, proline, cysteine, methionine, valine, leucine, isoleucine, tryptophan, phenylalanine, tyrosine, lysine, alanine, and glutamine. The amino acid can be used as a bulking agent, or as an anti-crystallization agent for drugs in the amorphous state, or as a crystal growth inhibitor for drugs in the crystalline state or as a wetting agent. Hydrophobic amino acids such as leucine, isoleucine, alanine, glycine, valine, proline, cysteine, methionine, phenylalanine, tryptophan are more likely to be effective as anticrystallization agents or crystal growth inhibitors. In addition, amino acids can serve to make the shell have a pH dependency that can be used to influence the pharmaceutical properties of the shell such as solubility, rate of dissolution or wetting.

Excipients, Bulking Agents

The drug particles are blended with one or more excipients particles. The term "excipient" refers to any non-active ingredient of the formulation intended to facilitate handling, stability, dispersibility, wettability, release kinetics, and/or parenteral administration of the pharmaceutical agent. The excipient may be a pharmaceutically acceptable carrier or a bulking agent as known in the art. The excipient may comprise a shell material, protein, amino acid, sugar or other carbohydrate, starch, lipid, or combination thereof. In one embodiment, the excipient is in the form of microparticles. In one embodiment, the excipient microparticles may have a volume average size between about 1 and 20 $\mu$m.

In one embodiment, the excipient in the methods and formulations described herein is a pre-processed excipient. A pre-processed excipient is one that initially cannot be readily handled in a dry powder form that is converted into a form suitable for dry powder processing (e.g., milling or blending). A preferred pre-processing process is described above. In preferred embodiments, the excipient of the pre-processed excipient comprises a liquid, waxy, non-crystalline compound, or other non-friable compound. In a preferred embodiment, the non-friable excipient comprises a surfactant, such as a waxy or liquid surfactant. By "liquid," it is meant that the material is a liquid at ambient temperature and pressure conditions (e.g., 15-25 °C and atmospheric pressure). Examples of such surfactants include docusate sodium (DSS) and polysorbates. In a preferred embodiment, the surfactant is a Tween or other hydrophilic non-ionic surfactant. The pre-processed excipient further includes at least one bulking agent. In preferred embodiments, the bulking agent used in the methods and formulations described herein comprises at least one sugar, sugar alcohol, starch, amino acid, or combination thereof. Examples of suitable bulking agents include lactose, sucrose, maltose, mannitol, sorbitol, trehalose, galactose, xylitol, erythritol, and combinations thereof.

In one particular embodiment of the methods described herein, a saccharide (e.g., mannitol) and a surfactant (e.g., TWEEN™ 80) are blended in the presence of water and the water is then removed by spray-drying or lyophilization, yielding a pre-processed excipient of saccharide and surfactant. The pre-processed saccharide / surfactant blend is then blended with microparticles formed of or including an API.

Representative amino acids that can be used as excipients include both naturally occurring and non-naturally occurring amino acids. The amino acids can be hydrophobic or hydrophilic and may be D amino acids, L amino acids or racemic mixtures. Amino acids which can be used include glycine, arginine, histidine, threonine, asparagine, aspartic acid, serine, glutamate, proline, cysteine, methionine, valine, leucine, isolcucine, tryptophan, phenylalanine, tyrosine, lysine, alanine, and glutamine. The amino acid can be used as a bulking agent, as a wetting agent, or as a crystal growth inhibitor for drugs in the crystalline state. Hydrophobic amino acids such as leucine, isoleucine, alanine, glycine, valine, proline, cysteine, methionine, phenylalanine, tryptophan are more likely to be effective as crystal growth inhibitors. In addition, amino acids can serve to make the matrix have a pH dependency that can be used to influence the pharmaceutical properties of the matrix, such as solubility, rate of dissolution, or wetting.

Examples of excipients include surface active agents, dispersants, osmotic agents, diluents. Examples include sodium desoxycholate; sodium dodecylsulfate; polyoxyethylene sorbitan fatty acid esters, e.g., polyoxyethylene 20 sorbitan monolaurate (TWEEN™ 20), polyoxyethylene 4 sorbitan monolaurate (TWEEN™ 21), polyoxyethylene 20 sorbitan monopalmitate (TWEEN™ 40), polyoxyethylene 20 sorbitan monooleate (TWEEN™ 80); polyoxyethylene alkyl ethers, e.g., polyoxyethylene 4 lauryl ether (BRIJ™ 30), polyoxyethylene 23 lauryl ether (BRIJ™ 35), polyoxyethylene 10 oleyl ether (BRIJ™ 97); polyoxyethylene glycol esters, e.g., poloxyethylene 8 stearate (MYRJ™ 45), poloxyethylene 40 stearate (MYRJ™ 52); Tyloxapol; Spans; and mixtures thereof.

The invention can further be understood with reference to the following non-limiting examples.

## Examples

Materials were obtained from the following sources: celecoxib and oxcarbazepine (Onbio, Richmond Hill, Ontario, Canada), mannitol (Spectrum Chemicals, New Brunswick, NJ; or Pearlitol 100SD from Roquette America Inc., Keokuk, IA), TWEEN™ 80 (Spectrum Chemicals, New Brunswick, NJ), and Plasdone-C15 (International Specialty Products, Wayne, NY). The TWEEN™ 80 is hereinafter referred to as "Tween80."

A TURBULA™ inversion mixer (model: T2F) was used for blending. A Hosokawa Alpine Aeroplex Spiral Jet Mill (model: 50AS) or a Fluid Energy Aljetjet mill were used. Dry nitrogen gas, as the injector and grinding gases, was used for milling. In the studies, the dry powder was fed manually into the jet mill, and hence the powder feed rate was not constant. Although the powder feeding was manual, the feed rate was calculated to be approximately 1 to 5 g/min. for all of the studies. Feed rate is the ratio of total material processed in one batch to the total batch time. Particle size measurement of the jet milled samples, unless otherwise indicated, was conducted using a Coulter Multisizer II with a 50 μm aperture.

### Example 1: Jet Milling a Blend of PLGA Microparticles with Pre-processed Excipient Particles Comprising Tween80 and Mannitol

Blending was conducted in two steps: a first step in which an excipient was pre-processed into a dry powder form and a second step in which the particles (representing particles of a pharmaceutical agent) were combined with the particles of pre-processed excipient. In the first step, mannitol and Tween80 were blended in liquid form, wherein 500 mL of Tween80/mannitol vehicle was prepared from Tween80, mannitol, and water. The vehicle was frozen and then subjected to vacuum drying, yielding the pre-processed excipient: a powder comprised of Tween80 homogeneously dispersed with the mannitol. In the second step, poly(lactide-co-glycolide) (50:50) ("PLGA") microparticles (which represented the pharmaceutical agent particles) were combined with the pre-processed mannitol/Tween80 and mixed in a tumbler mixer to yield a dry blended powder. The PLGA microparticles had an Xn = 2.83 micron and Xv = 8.07 micron. The dry blended powder was then fed manually into a jet mill, operated at three different sets of operating conditions. The resulting milled blend samples were analyzed for particle size. For comparison, a control sample (blended but not jet milled) was similarly analyzed. The Coulter Multisizer II results are shown in **Table 1**.

**Table 1: Results of Particle Size Analysis**

| Sample | Number Avg. Particle Size, $X_n$ (μm) | Volume Avg. Particle Size, $X_v$ (μm) |
|---|---|---|
| Control | 2.78 | 8.60 |
| 2.1 | 1.98 | 4.52 |
| 2.3 | 1.99 | 4.11 |
| 2.3 | 1.93 | 3.37 |

The results demonstrate the advantage to dispersibility (as assessed by volume mean (Xv), with a smaller Xv being an indicator of decreased agglomerates) offered by milled blend formulations.

### Example 2: Jet Milling of a Blend of Celecoxib with Pre-processed Excipient Particles Comprising Tween80, Plasdone-C15, and Mannitol

Two blends were made containing celecoxib: mannitol: Tween80: Plasdone-C15 in a 10:10:1:1 ratio. Sample 2a was made by jet milling a blend of celecoxib, mannitol (Pearlitol 100SD), Tween80, and Plasdone-C15 directly (no pre-processing of the excipients). Sample 2b was made by jet milling a blend of celecoxib and pre-processed mannitol/Tween80/Plasdone-C 15. The mannitol and the Tween80 were pre-processed, at a ratio of 10:1, by dissolution in water (85.2 g mannitol and 8.54 g Tween80 in 749 g water) followed by freezing and lyophilization.

Each sample was blended using a Turbula mixer, to produce a dry blended powder. The dry blended powder was then fed manually into a Fluid Energy Aljetjet mill. Observations were made regarding the processing ease of the milling, and the observations arc described in **Table 2.** The material made with pre-processed excipient was easier to mill than the material made with the non-preprocessed excipient.

**Table 2: Milling Observations Related to Ease of Processing**

| Sample | Milling Comment |
|---|---|
| 2a: Jet Milled Blend of Celecoxib and Non-preprocessed excipients | The mill clogged many times. Near the gasket of the jet mill, many aggregates (like granules) were observed. |
| 2b: Jet Milled Blend of Celecoxib and Preprocessed excipients | The mill clogged a few times. |

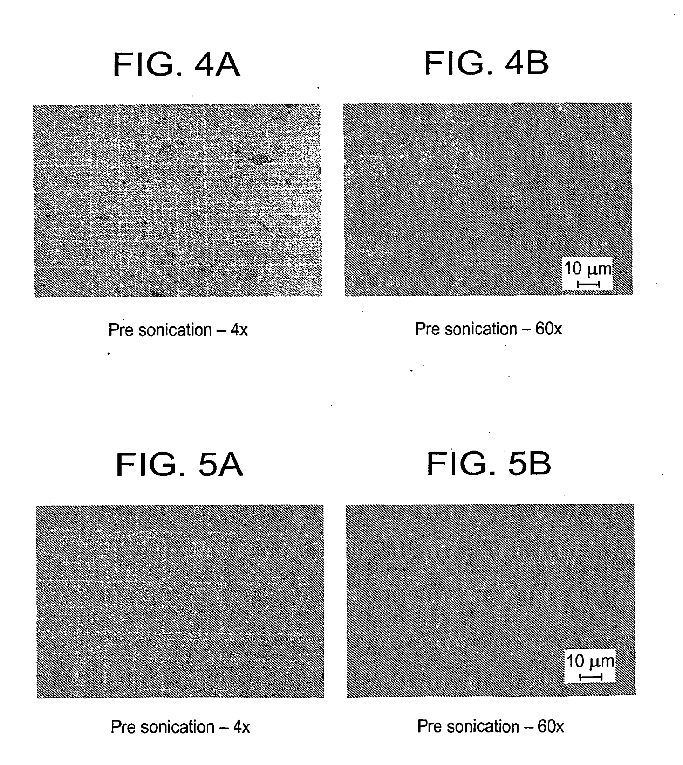

The resulting milled blends of Sample 2a and Sample 2b were reconstituted with water and examined by microscopy. There were agglomerates observed in the formulation containing non-lyophilized mannitol/Tween80. However, large agglomerates were not visible for the material that contained lyophilized mannitol/Tween80/PVP, indicating that pre-processing of the Tween80 excipient resulted in improved dispersal, as shown in **FIGS. 4A-B** (Sample 2a) and **FIG. 5A-B**(Sample 2b).

### Example 3: Microparticle Dispersibility Comparison of Reconstituted Celecoxib Parenteral Blend Formulations with Surfactant in the Blend

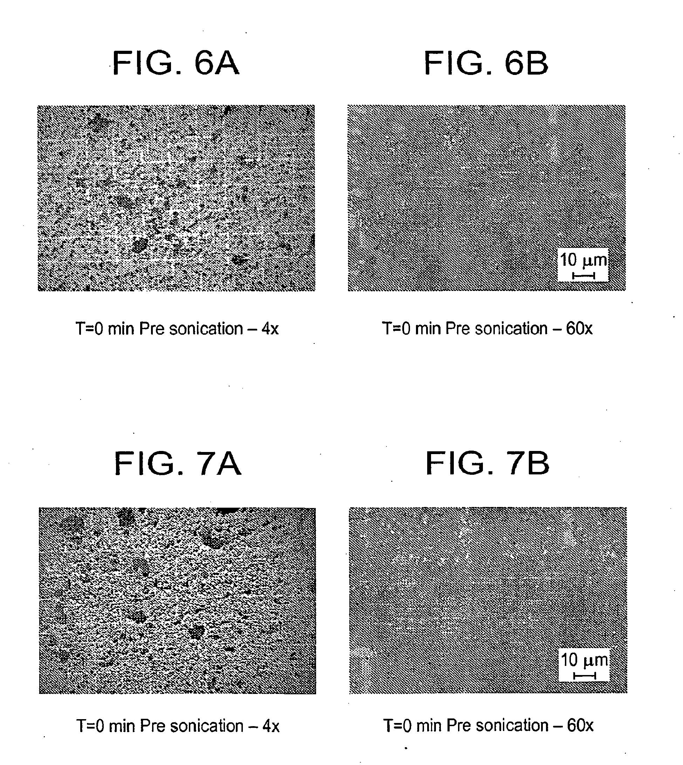

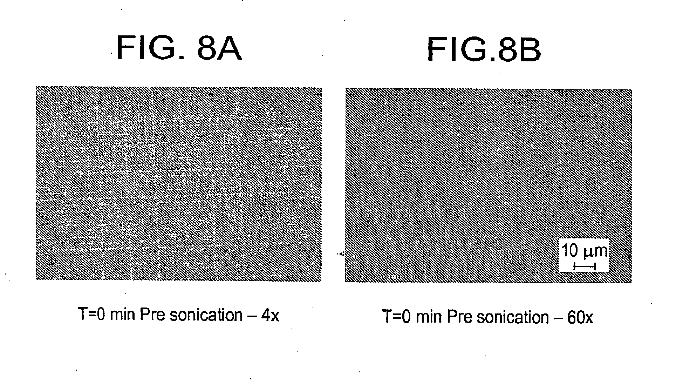

A dry powder blend formulation was prepared by one of three different processes and then reconstituted in water. The dry powder blend consisted of celecoxib, mannitol, Plasdone-C15, and Tween80 at a ratio of 5:10:1:1. The mannitol (Pearlitol 100SD) and the Tween80 were pre-processed, at a ratio of 10:1, by dissolution in water (18 g mannitol and 1.8 g Tween80 in 104 mL water) followed by freezing at -80 °C and lyophilization. The three processes compared were (1) blending the celecoxib and excipient particles without milling, (2) separately milling the cel ecoxib particles and then blending the milled particles with excipients, or (3) blending the celecoxib and excipient particles and then milling the resulting blend. The resulting blends were reconstituted in water using shaking, and analyzed by light scattering using an LS230 Laser Diffraction Particle Size Analyzer (Beckman Coulter, Fullerton, CA). The particles' sizes from each of the three processes were compared. The size results arc shown in **Table 3**, along with visual evaluations of the quality of the suspensions. **FIGS. 6A-B** show the microscopy results of reconstituted celecoxib from a blend of excipient particles and celecoxib particles (Process 1). **FIGS. 7A-B** show the microscopy results of reconstituted celecoxib from a blend of excipient particles and milled celecoxib particles (Process 2). **FIGS. 8A-B** show the microscopy results of reconstituted celecoxib from a jet milled blend of excipient particles and celecoxib particles (Process 3).

**Table 3: Results of Particle Size Analysis and Observations Following Reconstitution**

| Sample | LS230 Particle Size Analysis T = 0 Post Reconstitution | | Visual Evaluation of Suspension Post Reconstitution | |
|---|---|---|---|---|
| | Volume mean ($\mu$m) | % < 90 ($\mu$m) | T = 0 | T = 60 min |
| Celecoxib Particles Blended | 56.27 | 156.95 | Fine suspension with many small macroparticles | Fine suspension with many small macroparticles |
| Blend of Jet Milled Celecoxib Particles | 58.98 | 153.08 | Fine suspension with many small macroparticles | Fine suspension with many small macroparticles |
| Jet Milled Blend of Celecoxib Particles | 5.45 | 9.12 | Fine suspension with very few small macroparticles | Fine Suspension |

These results strongly indicate that the processing method impacts the resulting suspension quality. The results also indicate the advantages offered by milled blend formulations as compared to the formulations made by the other methods.

Jet milling of blended celecoxib particles led to a powder which was better dispersed, as indicated by the resulting fine suspension with a few macroscopic particles. This suspension was better than the suspensions of the unprocessed celecoxib microparticles and the blended celecoxib microparticles.

The light microscope images (**FIGS. 6-8**) of the suspensions indicate no significant change to individual particle morphology, just to the ability of the individual particles to disperse as indicated by the more uniform size and increased number of suspended microparticles following both blending and jet milling as compared to the two other microparticle samples.

**Example 4: Microparticle Dispersibility Comparison of Reconstituted Celecoxib Parenteral Blend Formulations Without Surfactant in the Blend**

A dry powder blend formulation not containing a surfactant was prepared by one of three different processes and then reconstituted in a vehicle containing a surfactant. The blend included celecoxib and mannitol (Pearlitol 100SD) in a ratio of 5:10. The three processes compared were (1) blending the celecoxib and mannitol particles without milling, (2) separately milling the celecoxib particles and then blending the milled particles with mannitol particles, or (3) blending the celecoxib and mannitol particles and then milling the resulting blend. The resulting blends were reconstituted by shaking with water containing 5.46 mg/mL Tween80 and 5.46 mg/mL Plasdone C15. The resulting suspensions were analyzed by light scattering using an LS230. The particles sizes of were compared for the three processes, and results are shown below in Table 4.

**Table 4: Results of Particle Size Analysis**

| Sample | LS230 Particle Size analysis T = 0 Post Reconstitution | |
| --- | --- | --- |
| | Volume mean ($\mu$m) | % < 90 ($\mu$m) |
| Celecoxib Particles Blended | 12.07 | 20.73 |
| Blend of Jet Milled Celecoxib Particles | 13.08 | 32.67 |
| Jet Milled Blend of Celecoxib Particles | 5.49 | 9.79 |

These results strongly indicate that the processing method impacts the suspension quality. In addition, the results demonstrate the advantages that may be obtained from milled blend formulations as compared to the formulations made by the other methods. The results also indicate that a surfactant can be added in the vehicle for reconstitution for parenteral administration rather than in the dry blend, which may be particularly advantageous in certain formulations and manufacturing processes.

**Example 5: Jet Milling of a Blend of Oxcarbazepine with Pre-processed Excipient Particles of Tween80 and Mannitol**

A blend was made containing oxcarbazepine: mannitol: Tween80 in a 10 : 5 : 0.273 ratio. The product was made by jet milling a blend of oxcarbazepine and pre-processed mannitol/Tween80. A combination of mannitol (Pearlitol 100SD) and Tween80 were pre-processed, at a ratio of 5 : 0.273, by dissolution in water (10 g mannitol and 0.546 g Tween80 in 60 g water) followed by freezing and lyophilization.

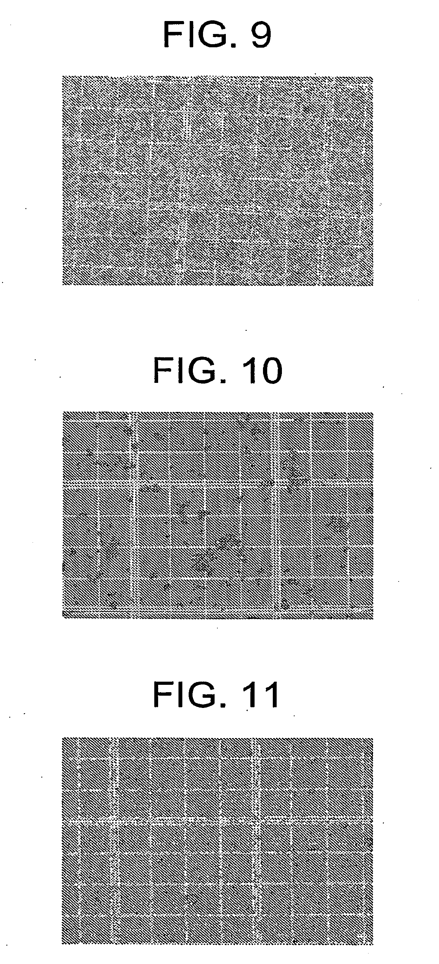

The blend was blended using a Turbula mixer, to produce a dry blended powder. The dry blended powder was then fed manually into a Fluid Energy Aljet jet mill. The resulting milled blend was reconstituted with water and examined by microscopy, revealing a well dispersed suspension of microparticles (**FIG. 9**).

**Example 6: Microparticle Dispersibility of an Oxcarbazepine Parenteral Blend Formulation Without Surfactant in the Blend**

A blend was made containing oxcarbazepine: mannitol: in a 10 : 5 ratio. The product was made by jet milling a blend of oxcarbazepine and mannitol. The blend was blended using a Turbula mixer, to produce a dry blended powder. The dry blended powder was then fed manually into a Fluid Energy Aljet jet mill. The resulting milled blend was reconstituted with either water or water containing 5.46 mg/mL Tween80 and examined by microscopy.

The sample reconstituted with water only revealed a poorly dispersed suspension of microparticles (**FIG. 10**), whereas the sample reconstituted with water containing Tween80 revealed a well dispersed suspension of microparticles (**FIG. 11**). These results indicate that a surfactant is needed to create a well dispersing suspension of oxcarbazepine, but the surfactant does not have to be a component of the blend prior to jet milling.

These results indicate that well dispersing suspensions of oxcarbazepine appropriate for parenteral administration can be made using milled blend formulations.

Publications cited herein and the materials for which they are cited are specifically incorporated by reference. Modifications and variations of the methods and devices described herein will be obvious to those skilled in the art from the foregoing detailed description. Such modifications and variations are intended to come within the scope of the appended claims.

**Claims**

1. A method for making a pharmaceutical formulation for parenteral administration, comprising the steps of:

   a) providing particles which comprise a pharmaceutical agent;
   b) dry blending the particles with particles of at least one bulking agent to form a first powder blend, which does not include a surfactant;
   c) milling the first powder blend to form a milled blend which comprises microparticles or nanoparticles of the pharmaceutical agent; and
   d) reconstituting the milled blend with a liquid vehicle suitable for parenteral administration, wherein the vehicle comprises at least one surfactant.

2. The method of claim 1, wherein the vehicle or the particles further comprises a polymer.

3. A method for making a pharmaceutical formulation for parenteral administration, comprising the steps of:

   a) providing particles which comprise a pharmaceutical agent; and
   b) dry blending the particles of step a) with particles of a pre-processed excipient to form a dry powder blend which does not include a surfactant, wherein the pre-processed excipient is prepared by

      i) dissolving a bulking agent and at least one non-friable excipient in a solvent to form an excipient solution, and
      ii) removing the solvent from the excipient solution to form the pre-processed excipient in dry powder form, wherein the dry powder blend can be reconstituted in an aqueous vehicle suitable for parenteral administration.

4. The method of claim 3, further comprising c) reconstituting the dry powder blend with a vehicle suitable for parenteral administration.

5. The method of claim 3, wherein the particles of step a), the particles of pre-processed excipient, or both, are either microparticles or nanoparticles.

6. The method of claim 3, wherein the non-friable excipient comprises a liquid, waxy, or non-crystalline compound.

7. The method of claim 3, wherein the non-friable excipient comprises a polyvinylpyrrolidone, a polyoxyethylenc sorbitan fatty acid ester, or a combination thereof.

8. The method of any one of claims 1 to 7, wherein the bulking agent comprises at least one sugar, sugar alcohol, starch, amino acid, or combination thereof, and is preferably selected from the group consisting of lactose, sucrose, maltose, mannitol, sorbitol, trehalose, galactose, xylitol, erythritol, and combinations thereof.

9. The method of claim 3, wherein the step of removing the solvent comprises spray-drying or lyophilization.

10. The method of claim 3, further comprising c) milling the dry powder blend to form a milled pharmaceutical formulation blend, which comprises microparticles or nanoparticles of the pharmaceutical agent.

11. The method of claim 10, further comprising d) reconstituting the dry powder blend with a vehicle suitable for parenteral administration.

**12.** The method of claim 1 or 10, wherein the milling of step c) comprises jet milling.

**13.** The method of any one of claims 1 to 12, wherein the particles of step a) are microparticles.

**14.** A pharmaceutical formulation for parenteral administration obtainable by the method of any one of claims 1 to 13.

**15.** A parenteral dosage form of a pharmaceutical formulation according to claim 14 comprising:

a liquid suspension of a milled blend of microparticles or nanoparticles of a pharmaceutical agent dry blended with particles of at least one excipient, dispersed in a pharmaceutically acceptable liquid vehicle for injection,

wherein the milled blend does not include a surfactant and the pharmaceutically acceptable liquid vehicle comprises an aqueous solution of a surfactant.

**16.** The method of any one of claims 1 to 13 or the parenteral dosage form of claim 15, wherein the pharmaceutical agent has a solubility in water of less than 10 mg/mL at 25°C.

**17.** The parenteral dosage form of claim 15, wherein the excipient particles comprise at least one sugar, sugar alcohol, starch, amino acid, or combination thereof.

**18.** The parenteral dosage form of claim 15, wherein the excipient particles include a pre-processed excipient which comprises a bulking agent and at least one non-friable excipient.

**19.** The parenteral dosage form of claim 15, wherein the microparticles or nanoparticles of a pharmaceutical agent further comprise a polymer.

**20.** A parenteral dosage form of a therapeutic or prophylactic formulation, which comprises:

a milled blend of
microparticles or nanoparticles of a therapeutic or prophylactic agent which has a solubility in water of less than 10 mg/mL at 25°C and
particles of at least one excipient comprising at least one sugar, sugar alcohol, starch, amino acid, or combination thereof;
a pharmaceutically acceptable liquid vehicle for injection in which the milled blend is dispersed; and

wherein (i) the excipient particles are pre-processed to comprise a surfactant, or (ii) the liquid vehicle comprises an aqueous solution of a surfactant, or (iii) both (i) and (ii).

**21.** The pharmaceutical formulation of claim 14 or the parenteral dosage form of any one of claims 15 to 20, wherein the pharmaceutical agent comprises oxcarbazepine.


**Patentansprüche**

**1.** Verfahren zur Herstellung einer pharmazeutischen Zusammensetzung für die parenterale Verabreichung, umfassend die Schritte:

(a) Bereitstellen von Partikeln, die ein pharmazeutisches Mittel umfassen;
(b) Trockenvermischen der Partikel mit Partikeln von zumindest einem Füllstoff, um eine erste Pulvermischung zu bilden, die kein Tensid umfasst;
(c) Mahlen der ersten Pulvermischung, um eine gemahlene Mischung zu bilden, die Mikropartikel oder Nanopartikel des pharmazeutischen Mittels umfasst; und
(d) Wiederaufbauen der gemahlenen Mischung mit einem flüssigen Vehikel, das für die parenterale Verabreichung geeignet ist, worin das Vehikel zumindest ein Tensid umfasst.

**2.** Verfahren gemäss Anspruch 1, worin das Vehikel oder die Partikel ferner ein Polymer umfassen.

**3.** Verfahren zur Herstellung einer pharmazeutischen Formulierung für die parenterale Verabreichung, umfassend die Schritte:

(a) Bereitstellen von Partikeln, die ein pharmazeutisches Mittel umfassen; und
(b) Trockenvermischen der Partikel aus Schritt (a) mit Partikeln eines vorverarbeiteten Exzipienten, um eine trockene Pulvermischung zu bilden, die kein Tensid umfasst, worin der vorverarbeitete Exzipient hergestellt ist durch

(i) Auflösen eines Füllstoffs und eines nicht-bröckeligen Exzipienten in einem Lösungsmittel, um eine Exzipientenlösung zu bilden, und
(ii) Entfernen des Lösungsmittels von der Exzipientenlösung, um den vorverarbeiteten Exzipienten in Trockenpulverform zu bilden,
worin die trockene Pulvermischung in einem wässrigen Vehikel, das für die parenterale Verabreichung geeignet ist, wiederaufgebaut werden kann.

**4.** Verfahren gemäss Anspruch 3, ferner umfassend (c) Wiederaufbauen der trockenen Pulvermischung mit einem Vehikel, das für die parenterale Verabreichung geeignet ist.

**5.** Verfahren gemäss Anspruch 3, worin die Partikel aus Schritt (a), die Partikel des vorverarbeiteten Exzipienten oder beide entweder Mikropartikel oder Nanopartikel sind.

**6.** Verfahren gemäss Anspruch 3, worin der nichtbröckelige Exzipient eine flüssige, wachsartige oder nicht-kristalline Verbindung umfasst.

**7.** Verfahren gemäss Anspruch 3, worin der nichtbröckelige Exzipient ein Polyvinylpyrrolidon, einen Polyoxyethylensorbitan-Fettsäureester oder eine Kombination hiervon umfasst.

**8.** Verfahren gemäss irgendeinem der Ansprüche 1 bis 7,
worin der Füllstoff zumindest einen Zucker, Zuckeralkohol, Stärke, Aminosäure oder eine Kombination hiervon umfasst und bevorzugt ausgewählt ist aus der Gruppe, bestehend aus Lactose, Saccharose, Maltose, Mannitol, Sorbitol, Trehalose, Galactose, Xylitol, Erythritol und Kombinationen hiervon.

**9.** Verfahren gemäss Anspruch 3, worin der Schritt zum Entfernen des Lösungsmittels Sprühtrocknen oder Gefriertrocknen umfasst.

**10.** Verfahren gemäss Anspruch 3, ferner umfassend (c) Mahlen der trockenen Pulvermischung, um eine gemahlene Mischung einer pharmazeutischen Formulierung zu bilden, die Mikropartikel oder Nanopartikel des pharmazeutischen Mittels umfasst.

**11.** Verfahren gemäss Anspruch 10, ferner umfassend (d) Wiederaufbauen der trockenen Pulvermischung mit einem Vehikel, das für die parenterale Verabreichung geeignet ist.

**12.** Verfahren gemäss Anspruch 1 oder 10, worin das Mahlen von Schritt (c) ein Strahlmahlen umfasst.

**13.** Verfahren gemäss irgendeinem der Ansprüche 1 bis 12,
worin die Partikel aus Schritt (a) Mikropartikel sind.

**14.** Pharmazeutische Formulierung für die parenterale Verabreichung, erhältlich durch ein Verfahren gemäss irgendeinem der Ansprüche 1 bis 13.

**15.** Parenterale Dosierungsform einer pharmazeutischen Formulierung gemäss Anspruch 14, umfassend:

eine flüssige Suspension einer gemahlenen Mischung von Mikropartikeln oder Nanopartikeln eines pharmazeutischen Mittels, trockenvermischt mit Partikeln von zumindest einem Exzipienten, dispergiert in einem pharmazeutisch akzeptablen, flüssigen Vehikel für die Injektion,

worin die gemahlene Mischung kein Tensid umfasst und das pharmazeutisch akzeptable flüssige Vehikel eine wässrige Lösung eines Tensids umfasst.

**16.** Verfahren gemäss irgendeinem der Ansprüche 1 bis 13 oder parenterale Dosierungsform gemäss Anspruch 15, worin das pharmazeutische Mittel eine Löslichkeit in Wasser von weniger als 10 mg/ml bei 25°C aufweist.

**17.** Parenterale Dosierungsform gemäss Anspruch 15, worin die Exzipientenpartikel zumindest einen Zucker, Zuckeralkohol, Stärke, Aminosäure oder Kombinationen hiervon umfassen.

**18.** Parenterale Dosierungsform gemäss Anspruch 15, worin die Exzipientenpartikel einen vorverarbeiteten Exzipienten umfassen, der einen Füllstoff und zumindest einen nicht-bröckeligen Exzipienten umfasst.

**19.** Parenterale Dosierungsform gemäss Anspruch 15, worin die Mikropartikel oder Nanopartikel eines pharmazeutischen Mittels ferner ein Polymer umfassen.

**20.** Parenterale Dosierungsform einer therapeutischen oder prophylaktischen Formulierung, die umfasst:

eine gemahlene Mischung von
Mikropartikeln oder Nanopartikeln eines therapeutischen oder prophylaktischen Mittels, das eine Wasserlöslichkeit von weniger als 10 mg/ml bei 25°C aufweist, und
Partikeln von zumindest einem Exzipienten, der zumindest einen Zucker, Zuckeralkohol, Stärke, Aminosäure oder eine Kombination hiervon umfasst;
ein pharmazeutisch akzeptables flüssiges Vehikel für die Injektion, worin die gemahlene Mischung dispergiert ist; und

worin (i) die Exzipientenpartikel vorverarbeitet sind, so dass sie ein Tensid umfassen oder (ii) das flüssige Vehikel eine wässrige Lösung eines Tensids umfasst, oder (iii) sowohl (i) und (ii).

**21.** Pharmazeutische Formulierung gemäss Anspruch 14 oder parenterale Dosierungsform gemäss irgendeinem der Ansprüche 15 bis 20, worin das pharmazeutische Mittel Oxcarbazepin umfasst.

**Revendications**

**1.** Procédé de préparation d'une formulation pharmaceutique destinée à une administration parentérale, comprenant les étapes consistant à :

a) fournir des particules qui comprennent un agent pharmaceutique ;
b) mélanger à sec les particules avec des particules d'au moins un diluant pour former un premier mélange pulvérulent, qui ne contient pas de tensioactif ;
c) broyer le premier mélange pulvérulent pour former un mélange broyé qui comprend des microparticules ou nanoparticules de l'agent pharmaceutique ; et
d) réhydrater le mélange broyé avec un milieu liquide approprié pour l'administration parentérale, dans lequel le milieu comprend au moins un tensioactif.

**2.** Procédé selon la revendication 1, dans lequel le milieu ou les particules comprennent en outre un polymère.

**3.** Procédé de préparation d'une formulation pharmaceutique destinée à une administration parentérale, comprenant les étapes consistant à :

a) fournir des particules qui comprennent un agent pharmaceutique ; et
b) mélanger à sec les particules de l'étape a) avec des particules d'un excipient prétraité pour former un mélange de poudre sèche qui ne contient pas de tensioactif,

dans lequel l'excipient prétraité est préparé par :

i) dissolution d'un diluant et d'au moins un excipient non-friable dans un solvant pour former une solution d'excipient, et
ii) élimination du solvant de la solution d'excipient pour former l'excipient prétraité sous forme d'une poudre sèche, dans lequel le mélange de poudre sèche peut être réhydraté dans un milieu aqueux approprié pour l'administration parentérale.

**4.** Procédé selon la revendication 3, comprenant en outre c) la réhydratation du mélange de poudre sèche avec un milieu approprié pour l'administration parentérale.

**5.** Procédé selon la revendication 3, dans lequel les particules de l'étape a), les particules de l'excipient prétraité, ou les deux types de particules, sont soit des microparticules soit des nanoparticules.

**6.** Procédé selon la revendication 3, dans lequel l'excipient non-friable comprend un composé liquide, cireux, ou non-cristallin.

**7.** Procédé selon la revendication 3, dans lequel l'excipient non-friable comprend une polyvinylpyrrolidone, un ester d'acides gras de polyoxyéthylène sorbitane, ou une combinaison de ceux-ci.

**8.** Procédé selon l'une quelconque des revendications 1 à 7, dans lequel le diluant comprend au moins un sucre, un polyol, un amidon, un acide aminé, ou une combinaison de ceux-ci, et est de préférence choisi dans le groupe consistant en lactose, saccharose, maltose, mannitol, sorbitol, tréhalose, galactose, xylitol, érythritol, et des combinaisons de ceux-ci.

**9.** Procédé selon la revendication 3, dans lequel l'étape d'élimination du solvant comprend le séchage par pulvérisation ou la lyophilisation.

**10.** Procédé selon la revendication 3, comprenant en outre c) le broyage du mélange de poudre sèche pour former un mélange de formulation pharmaceutique broyé, qui comprend des microparticules ou nanoparticules de l'agent pharmaceutique.

**11.** Procédé selon la revendication 10, comprenant en outre d) la réhydratation du mélange de poudre sèche avec un milieu approprié pour l'administration parentérale.

**12.** Procédé selon la revendication 1 ou la revendication 10, dans lequel le broyage de l'étape c) comprend le broyage à jet.

**13.** Procédé selon l'une quelconque des revendications 1 à 12, dans lequel les particules de l'étape a) sont des microparticules.

**14.** Formulation pharmaceutique destinée à une administration parentérale pouvant être obtenue par le procédé selon l'une quelconque des revendications 1 à 13.

**15.** Forme posologique parentérale d'une formulation pharmaceutique selon la revendication 14, comprenant :

une suspension liquide d'un mélange broyé de microparticules ou nanoparticules d'un agent pharmaceutique mélangé à sec avec des particules d'au moins un excipient, dispersé dans un milieu liquide pharmaceutiquement acceptable pour injection,

dans laquelle le mélange broyé ne contient pas de tensioactif et le milieu liquide pharmaceutiquement acceptable comprend une solution aqueuse d'un tensioactif.

**16.** Procédé selon l'une quelconque des revendications 1 à 13 ou forme posologique parentérale selon la revendication 15, dans lesquels l'agent pharmaceutique a une solubilité dans l'eau de moins de 10 mg/ml à 25 °C.

**17.** Forme posologique parentérale selon la revendication 15, dans laquelle les particules d'excipient comprennent au moins un sucre, un polyol, un amidon, un acide aminé, ou une combinaison de ceux-ci.

**18.** Forme posologique parentérale selon la revendication 15, dans laquelle les particules d'excipient contiennent un excipient prétraité qui comprend un diluant et au moins un excipient non-friable.

**19.** Forme posologique parentérale selon la revendication 15, dans laquelle les microparticules ou nanoparticules d'un agent pharmaceutique comprennent en outre un polymère.

**20.** Forme posologique parentérale d'une formulation thérapeutique ou prophylactique, qui comprend :

un mélange broyé de

microparticules ou nanoparticules d'un agent thérapeutique ou prophylactique qui a une solubilité dans l'eau de moins de 10 mg/ml à 25 °C, et de

particules d'au moins un excipient comprenant au moins un sucre, un polyol, un amidon, un acide aminé, ou une combinaison de ceux-ci ;

un milieu liquide pharmaceutiquement acceptable pour injection dans lequel le mélange broyé est dispersé ; et

dans laquelle (i) les particules d'excipient sont prétraitées pour comprendre un tensioactif, ou (ii) le milieu liquide comprend une solution aqueuse d'un tensioactif, ou (iii) les deux (i) et (ii).

21. Formulation pharmaceutique selon la revendication 14 ou forme posologique parentérale selon l'une quelconque des revendications 15 à 20, dans laquelle l'agent pharmaceutique comprend de l'oxcarbazépine.

# FIG. 1

Pharmaceutical Agent → Particle Production Process ← Optional Shell Material, Excipients

Bulking Agent Particles → Blending Process ← (from Particle Production Process)

Blending Process → Milling Process → Reconstitution Process ← Liquid Media

Reconstitution Process → Parental Dosage Form of Pharmaceutical Agent Particles

EP 1 973 527 B1

FIG. 2

EP 1 973 527 B1

Pharmaceutical
Agent

Excipient

| Particle Production Process |

| Pre-processing |

Bulking
Agent

Microparticles

Pre-processed
Excipient

Liquid Media

| Blending Process |

| Jet Milling Process |

| Reconstitution Process |

Suspension of
Microparticles
Parenteral dosage
Form

# FIG. 3

Solvent

Difficult-to-Process
Excipient

Bulking
Agent

Mixing/Dissolution
Process

Solution

Solvent
Removal
Process

Solvent

Dry Powder Form of
Excipient/Bulking
Agent

# FIG. 4A

Pre sonication – 4x

# FIG. 4B

10 µm

Pre sonication – 60x

# FIG. 5A

Pre sonication – 4x

# FIG. 5B

10 µm

Pre sonication – 60x

## FIG. 6A

T=0 min Pre sonication – 4x

## FIG. 6B

10 μm

T=0 min Pre sonication – 60x

## FIG. 7A

T=0 min Pre sonication – 4x

## FIG. 7B

10 μm

T=0 min Pre sonication – 60x

## FIG. 8A

T=0 min Pre sonication – 4x

## FIG.8B

10 μm

T=0 min Pre sonication – 60x

## FIG. 9

## FIG. 10

## FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5853698 A, Straub
- US 5611344 A, Bernstein
- US 6395300 B, Straub
- US 6223455 B, Chickering III
- US 6143211 A, Mathiowitz
- US 6962006 B, Chickering III

### Non-patent literature cited in the description

- **Mathiowitz et al.** *J. Scanning Microscopy,* 1990, vol. 4, 329
- **Beck et al.** *Fertil. Steril,* 1979, vol. 31, 545
- **Benita et al.** *J. Pharm. Sci.,* 1984, vol. 73, 1721
- **Mathiowitz et al.** *Reactive Polymers,* 1987, vol. 6, 275
- **Martindale.** The Extra Pharmacopoeia. The Pharmaceutical Press, 1993